# EUROPÄISCHE PATENTSCHRIFT

(11) **EP 1 214 406 B1**
(45) Veröffentlichungstag und Bekanntmachung des Hinweises auf die Patenterteilung: **27.08.2008**
(21) Anmeldenummer: 00958528.2
(22) Anmeldetag: 08.09.2000
(51) Int. Cl.: C12N 15/10, C12Q 1/68

(54) **VERFAHREN ZUM ANBINDEN VON NUKLEINSÄUREN AN EINE FESTPHASE**
METHOD FOR BINDING NUCLEIC ACIDS TO A SOLID PHASE
PROCEDE POUR LIER DES ACIDES NUCLEIQUES A UNE PHASE SOLIDE

(30) Priorität: 10.09.1999 DE 19943374
(43) Veröffentlichungstag der Anmeldung: 19.06.2002
(73) Patentinhaber: Max-Planck-Gesellschaft zur Förderung der Wissenschaften e.V., 80539 München (DE)
(72) Erfinder: RAUTH, Holger, D-10823 Berlin (DE); REINHARDT, Richard, D-14195 Berlin (DE); NORDHOFF, Eckard, D-10829 Berlin (DE)
(74) Vertreter: Dey, Michael
(86) Internationale Anmeldenummer: PCT/EP2000/008807
(87) Internationale Veröffentlichungsnummer: WO 2001/019980

(56) Entgegenhaltungen:
- EP-A- 0 389 063
- EP-A- 0 580 305
- US-A- 4 579 661
- US-A- 5 705 628

## Beschreibung

Die Erfindung betrifft Verfahren zum Anbinden bzw. Immobilisieren von Nukleinsäuren an eine Festphase sowie zum Aufreinigen der gebundenen Nukleinsäuren, wobei die Festphase mit hydrophilen und hydrophoben Gruppen belegt ist, oder/und eine hydrophile wasserhaltige Polymermatrix.

Für viele Arbeitstechniken ist es erforderlich, daß die eingesetzten Nukleinsäuren, insbesondere DNA, frei von störenden Begleitsubstanzen sind. Mit herkömmlichen Verfahren erhaltene Nukleinsäuren müssen deshalb vor einer Weiterverwendung in den meisten Fällen aufgereinigt werden. Beispielsweise müssen vor einer Sequenzierung von PCR (Polymerasekettenreaktion) -Produkten unerwünsche Nebenprodukte, überschüssiger Primer, nicht eingebaute Nukleotide und Salze des Reaktionspuffers abgetrennt werden, da sie die Sequenzierreaktion stören könnten. Auch bei einer Vervielfältigung von DNA mit Hilfe von Zellen muß vor der Weiterverarbeitung der gewünschten DNA eine Aufreinigung erfolgen, bei der das Zelldebris nach einer Lyse abgetrennt wird. Die gewünschte DNA muß dann vor der Verwendung, z.B. für einen Restriktionsverdau oder eine Sequenzierreaktion, von Verunreinigungen, wie RNA, Proteinen, Salzen und dgl. befreit werden. Auch für eine quantitative Bestimmung von Nukleinsäuren, beispielsweise über UV-Absorptionsmessungen, ist zunächst eine Aufreinigung erforderlich, da für eine fehlerfreie Bestimmung die verwendete Nukleinsäurelösung frei von anderen Bestandteilen sein muß, die im gleichen Wellenbereich wie das gewünschte Produkt absorbieren, beispielsweise RNA, Primer, Nukleotide und dgl. Auch bei einer Konzentrationsbestimmung durch fluorimetrische Messungen müssen aufgereinigte Nukleinsäuren verwendet werden, damit es nicht zu unspezifischer, das Ergebnis verfälschender Fluoreszenz kommt.

Bei massenspektrometrischen Untersuchungen von Nukleinsäuren, insbesondere DNA, beispielsweise mit MALDI-MS (Matrix assisted Laser Desorption/lonisation-Mass Spectrometry; Matrix-unterstützte Laserdesorption/lonisations-Massenspektrometrie) ist es nötig, daß die Probemoleküle weitgehend frei von Begleitstoffen, wie etwa Puffersubstanzen, Metallkationen, Primerüberschüssen, Peptiden, Lipiden, Detergentien und dgl., sind, welche die Analyse stören könnten. Weiterhin wird eine Nukleinsäure zur Durchführung einer MALDI-MS-Analyse vorteilhafterweise in die Ammoniumform überführt. Auf diese Weise lassen sich diskrete Analytsignale und ein gutes Signal/Rausch-Verhältnis erreichen, und die Diskriminierung des Probensignals durch Begleitstoffe wird vermieden.

Bei der Aufarbeitung von wenigen Proben kann die Aufreinigung zwar manuell mit aufwendigen Techniken erfolgen. Zur Bewältigung einer größeren Anzahl von Proben ist es jedoch erforderlich, ein geeignetes, technisch einfaches und kostengünstiges Aufreinigungsverfahren bereitzustellen, das automatisiert werden kann, um den geforderten Durchsatz zu bewältigen.

Bisher sind verschiedene Verfahren zur Aufreinigung von Nukleinsäuren bekannt. Bei der Reinigung über Säulen kommen unterschiedliche Techniken zum Einsatz. Beispielsweise wird beim QIAquickPCR Purification Kit von Qiagen DNA mit Hilfe eines speziellen Bindungspuffers an eine Silicamembran adsorbiert. Der Bindungspuffer bewirkt, daß nur DNA bestimmter Länge adsorbiert wird und überschüssiger Primer und Nukleotide abgetrennt werden können. Nach dem Waschen der DNA wird diese dann mit einem geeigneten Elutionsmittel von der Säule eluiert.

Bei der Ausschluß-Chromatographie wird eine flüssige Phase, welche gelöste DNA enthält, auf eine Gelmatrix gegeben, wobei die Makromoleküle in Abhängigkeit ihrer Größe verschieden tief in das Netzwerk der Matrix eindringen. Kleinere Moleküle dringen tiefer ein als größere Moleküle und werden somit länger auf der Säule zurückgehalten. Moleküle, die größer als die größten Poren der verwendeten gequollenen Gelmatrix sind, können die Gelkörner nicht durchdringen und wandern an diesen vorbei, so daß sie die Säule zuerst verlassen. Aufgrund der unterschiedlichen Größe von gewünschter DNA auf der einen Seite und Primern und Nukleotiden auf der anderen Seite, kann eine Aufreinigung erfolgen. Ein häufig verwendetes Material für die Gelmatrix ist Sephacryl von Pharmacia.

Bei den Säulentechniken wird das Eluat üblicherweise durch Zentrifugation erhalten, weshalb diese Techniken nur mit sehr hohem Aufwand automatisiert werden können. Damit sind Säulentechniken nicht für einen hohen Probendurchsatz geeignet. Darüber hinaus ist die Aufreinigung über Säulen mit einem komplexen apparativen Aufwand und damit mit hohen Kosten verbunden.

Ein weiteres Verfahren zur Aufreinigung von DNA ist die präparative Isolierung mittels Gelelektrophorese. Dabei werden die Moleküle nach ihrer Größe getrennt. Die gewünschte Bande mit den interessierenden Molekülen wird ausgeschnitten, und diese werden dann direkt aus dem Gel eluiert. Auch dieses Verfahren ist nur schwer zu automatisieren, wobei der limitierende Schritt das Ausschneiden der gewünschten Bande ist. Diese Methode konnte deshalb bisher nur für den manuellen Einsatz verwendet werden.

Ein weiteres Verfahren zur Aufreinigung von Nukleinsäuren ist die Magnetseparation mittels spezifischer Bindung der Nukleinsäuren an eine funktionalisierte Oberfläche. Bei einer spezifischen Bindung werden gezielt nur bestimmte DNA-Fragmente über hochaffine Wechselwirkungen oder kovalente Bindung an Partikel gebunden. Beispielsweise werden an mit immobilisiertem Streptavidin ausgestattete Magnetpartikel über hochaffine Wechselwirkungen biotinylierte Produkte gebunden. Neben der Verwendung von Oberflächen, die einen Partner eines spezifischen Bindepaares tragen, können auch Partikel verwendet werden, die an ihrer Oberfläche einen Primer tragen. Unter geeigneten Hybridisierungsbedingungen werden dann nur Fragmente mit zu diesem Primer komplementärer Sequenz gebunden. Diese Verfahren erfordern jedoch eine aufwendige Vorbereitung sowohl der Festphasen als auch der aufzureinigenden Moleküle, beispielsweise durch Derivatisierung und sind auf so vorbereitete Moleküle limitiert.

WO 94/11103 beschreibt solch ein Verfahren unter Verwendung von magnetisierbaren Polymerpartikeln, die auf ihrer Oberfläche spezifische Affinitätsliganden tragen.

EP 0 389 063 beschreibt ein Verfahren zur Isolierung von Nukleinsäuren aus einem nukleinsäurehaltigen Ausgangsmaterial sowie ein Testkit zur Amplifizierung der mittels des Verfahrens erhaltenen Nukleinsäuren. Das Verfahren umfasst das Inkontaktbringen des nukleinsäurehaltigen Ausgangsmaterials mit einer chaotropen Substanz und einer die Nukleinsäuren bindenden Festphase.

EP 0 885 958 beschreibt ein Verfahren zur Isolierung von DNA unter Verwendung von mindestens zwei verschiedenen Magnetpartikeln, die Partner eines spezifischen Bindepaares, beispielsweise Sonden, Biotin oder Streptavidin tragen.

US-Patent 4,579,661 beschreibt die Reinigung biologisch aktiver Substanzen mit Hilfe eines Systems mindestens zweier unmischbarer wässriger Phasen. Das Verfahren umfasst das Anbinden der Substanzen an Partikel, welche eine Affinität für jene Substanzen besitzen und sich nahezu quantitativ in einer der Phasen verteilen, sowie das anschließende Freisetzen der Substanzen von diesen Partikeln nach Abtrennung der partikelhaltigen Phase von der oder den anderen Phasen.

US-Patent 5,405,951 beschreibt ein Verfahren, bei dem DNA unter Verwendung von chaotropen Salzen an Silicaoberflächen gebunden wird. Chaotrope Salze sind jedoch gesundheitsgefährdend. Zudem ist bei dem im US-Patent 5,405,951 beschriebenen Verfahren ein Arbeiten bei erhöhter Temperatur erforderlich.

US-Patent 5,705,628 beschreibt ein Verfahren, bei dem DNA unter Verwendung eines Bindepuffers an magnetische Mikropartikel gebunden wird, die eine mit Carboxylgruppen ausgestattete Oberfläche aufweisen. Allerdings werden dort aufgrund der geringen Ausbeute relativ viele Partikel pro Probe benötigt und die Carboxylgruppen-Modifikation erlaubt nur die Verwendung von wenigen, speziellen Partikelsorten.

Aufgabe der Erfindung war es somit, ein Verfahren zur Immobilisierung/Anbindung bzw. Aufreinigung von Nukleinsäuren bereitzustellen, welches die Nachteile der im Stand der Technik bekannten Verfahren zumindest teilweise vermeidet und welches insbesondere eine einfache und kosteneffiziente Aufreinigung einer großen Anzahl von Proben ermöglicht.

Diese Aufgabe wird erfindungsgemäß gelöst durch ein Verfahren zum Anbinden von Nukleinsäuren an eine Festphase, welches dadurch gekennzeichnet ist, daß eine Nukleinsäuren enthaltende Lösung mit einer Festphase, die hydrophobe und hydrophile Gruppen auf der Oberfläche aufweist, in Gegenwart eines Salzes und Polyethylenglykols in Kontakt gebracht wird, wobei die Nukleinsäuren reversibel und sequenzunspezifisch über die hydrophoben Gruppen an die Oberfläche gebunden werden. Unter diesen Bedingungen binden die Nukleinsäuremoleküle an besagte Oberfläche und stehen somit für festphasengestützte Waschvorgänge bereit, während derer störende Substanzen effektiv abgetrennt werden können und bei Bedarf die Probenmoleküle in die für die Massenspektrometrie günstige Ammoniumform überführt werden können.

Mit dem erfindungsgemäßen Verfahren können Nukleinsäuren aus Lösungen isoliert werden, wobei unter dem Begriff Nukleinsäure auch deren Salze zu verstehen sind. Die Bindung der Nukleinsäuren an die Oberfläche der Festphase erfolgt bevorzugt reversibel und unspezifisch und ist somit nicht durch spezielle hochaffine Bindepaare, wie etwa Streptavidin/Avidin, oder auf Nukleinsäuren mit bestimmten Sequenzabschnitten begrenzt, die z.B. durch Hybridisierung an die Oberflächen gebunden werden. Die Bindung von Nukleinsäuren erfolgt erfindungsgemäß vielmehr sequenzunabhängig, so daß eine universelle Bindematrix für Nukleinsäuren erhalten wird. Das Verfahren ist technisch einfach durchzuführen und kann ohne großen Aufwand automatisiert werden, so daß ein hoher Probendurchsatz bei geringen Kosten möglich ist. Das Immobilisieren und auch das Eluieren der Nukleinsäuren von der Oberfläche kann bei Raumtemperatur durchgeführt werden. Weiterhin wurde überraschenderweise festgestellt, daß Nukleinsäuren mit hoher Ausbeute an besagte Oberflächen gebunden werden können. Dies hat zur Folge, daß bei dem erfindungsgemäßen Verfahren weniger Partikel pro Probe eingesetzt werden müssen als bei bekannten Verfahren, wodurch eine weitere Vereinfachung und Kostenverringerung erzielt werden kann.

Bevorzugt weist der mit der Lösung in Kontakt gebrachte Teil der verwendeten Festphase einen Anteil von ≥ 1 %, insbesondere ≥ 5 % bevorzugt ≥ 10 % und von ≤ 90 %, insbesondere ≤ 50 % und bevorzugt ≤ 30 % an hydrophoben chemischen Oberflächengruppen auf. Dabei ist zu beachten, daß ab einem gewissen Anteil an hydrophoben Oberflächengruppen, der für die jeweilige Festphase ohne weiteres vom Fachmann bestimmt werden kann, ein Verklumpen von Festphasenpartikeln in wäßriger Lösung auftritt, mit der Folge, daß die Festhase nicht mehr resuspendierbar ist.

Erfindungsgemäß bevorzugt werden kleine, insbesondere magnetische Partikel eingesetzt, die auf ihrer Oberfläche funktionelle, hydrophobe Gruppen, wie etwa Alkyl- oder Arylgruppen tragen, verwendet, woran Nukleinsäuren unspezifisch und reversibel gebunden werden können. Günstigerweise werden aus der Umkehrphasenchromatographie bekannte funktionelle Gruppen verwendet, da deren Immobilisierung und Handhabung gut verstanden und etabliert ist.

Es ist auch möglich, eine Festphase mit mehreren, voneinander z.B. durch inerte Bereiche abgegrenzten aktiven Oberflächenbereichen zu verwenden, um mehrere räumlich voneinander abgegrenzte Reagenzfelder bereitzustellen.

Besagte Oberfläche auf der Festphase kann, sofern nicht vorhanden, durch Derivatisierung oder durch Beschichtung bereitgestellt werden. Diese Verfahrensweise hat den Vorteil, daß das Material für die Festphase frei wählbar ist. Die hydrophoben chemischen Gruppen sind vorzugsweise organische Kohlenwasserstoff-enthaltende Gruppen und können cyclische, lineare oder/und verzweigte Strukturen aufweisen. Bevorzugt trägt die Oberfläche C₁-C₃₀-Alkylgruppen, oder C₅-C₃₀-Arylgruppen, insbesondere C₆-C₂₄-Alkylgruppen als funktionelle, hydrophobe Gruppen. Besonders bevorzugt werden die Alkylgruppen ausgewählt aus C₈-Alkyl, C₁₈-Alkyl und Mischungen davon, wobei Octadecyl-Liganden am meisten bevorzugt sind.

Neben hydrophoben Gruppen weist die Oberfläche weiterhin hydrophile chemische Gruppen, z.B. Hydroxyl- oder/und Oxidgruppen, auf, die ggf. bei einer der unten genannten Vor- bzw. Zwischenbeschichtungen bereits vorhanden sein können. Wie oben ausgeführt neigen insbesondere kleine Partikel mit ausschließlich hydrophober Oberfläche zum Verklumpen in wäßrigen Lösungen. Dieses Problem kann vermieden werden, indem den funktionellen hydrophoben Gruppen hydrophile Gruppen, insbesondere Hydroxylreste zur Seite gestellt werden. Die Hydroxylgruppen können anorganische Hydroxylgruppen, z.B. Kieselsäuregruppen, oder/und organische Hydroxylgruppen, z. B. Mono- oder Polysaccharide wie Agarose, umfassen. Weitere geeignete hydrophile Gruppen umfassen Carbonyl-, Carboxyl-, Ester-, Amino-, Thiol-, Sulfat-, Sulfonyl- und ähnliche Gruppen und Kombinationen solcher Gruppen. Auch Polyolderivate, wie etwa Polyalkylenglykolderivate können als hydrophile Gruppen eingesetzt werden.

Die Anordnung und das Verhältnis von hydrophoben und hydrophilen Gruppen in dem zur Anbindung der Nukleinsäuren vorgesehenen Bereich der Oberfläche wird so eingestellt, daß die jeweiligen Partikel in wäßriger Lösung gerade nicht mehr verklumpen, die Bindung der Nukleinsäure aber noch wirksam stattfindet. Die funktionellen Gruppen sowie deren Anordnung können vom Fachmann den jeweiligen Parametern, wie etwa der Partikelgröße, der Partikeldichte, dem Analyten etc. angepaßt ausgewählt werden. Es ist beispielsweise möglich, die hydrophilen Gruppen in von den hydrophoben Gruppen abgegrenzten, separaten Mikrobereichen aufzubringen. Bevorzugt ist jedoch eine "Mischoberfläche", in der die hydrophoben und hydrophilen Gruppen nebeneinander vorliegen. Die hydrophilen Gruppen können auch durch geeignet substituierte organische Moleküle, z.B. Hydroxy-substituierte Alkyle eingebracht werden. Ein Beispiel für eine bevorzugte Mischoberfläche ist eine Alkyl/OH-Oberfläche, insbesondere eine C₁₈-Alkyl/OH-Oberfläche.

Besagte Oberflächen können direkt oder mittels einer Vor- oder Zwischenbeschichtung auf die Festphase aufgebracht sein. Geeignete Vorbeschichtungen sind beispielsweise eine Polykieselsäurematrix oder/und eine Monosaccharidmatrix. Andere geeignete Vorbeschichtungen sind Partner eines spezifischen Bindepaares, beispielsweise Streptavidin/Avidin, auf die dann die eigentliche aktive Schicht aufgebracht wird. Die Bindung der hydrophoben oder/und hydrophilen Gruppen an die Festphase bzw. die Bindung der hydrophoben oder/und hydrophilen Beschichtung an die Vorbeschichtung und die Bindung der Vorbeschichtung an die Festphase kann z.B. kovalent, z.B. durch Veresterung, adsorptiv oder über hochaffine Wechselwirkungen erfolgen. Bei einer besonders bevorzugten Ausführungsform wird eine Vorbeschichtung bestehend aus Polykieselsäure und Monosaccharid auf eine Festphase, beispielsweise γ-Eisenoxidpartikel mit einem Durchmesser im Nanometer- oder Mikrometerbereich aufgebracht. Diese Vorbeschichtung wird dann mit hydrophoben Gruppen, insbesondere Alkylgruppen und ggf. auch mit hydrophilen Gruppen, ausgestattet. Besonders gute Ergebnisse wurden mit Partikeln erhalten, die 10 bis 15% Alkylgruppen, insbesondere Octadecylgruppen pro Kieselsäure-Matrix (mol/mol) und 0,2 ‰ Octadecylgruppen pro Monosaccharid-Einheit (mol/mol) aufweisen.

Als Festphase kann jede dem Fachmann bekannte Festphase verwendet werden, wie etwa Mikrotiterplatten, Gefäße, wie etwa Eppendorf-Gefäße, Greiner tubes, Nunc Röhrchen etc. Bevorzugt werden als Festphase Feststoffpartikel mit einem Durchmesser ≥ 1 nm bis ≤ 1 mm eingesetzt, wodurch eine günstige spezifische Oberfläche pro Gramm Partikel zugänglich ist. Das erfindungsgemäße Verfahren erlaubt den Einsatz verschiedenster Festphasenmaterialien. Bevorzugt wird als Festphasenmaterial Silica, ein Kunststoff wie etwa Polystyrol, oder ein magnetisches oder magnetisierbares Material verwendet und insbesondere γ-Eisenoxid.

Wenn eine magnetische Festphase verwendet wird, können weitere Vorteile erhalten werden. Eine Magnetseparation ist relativ einfach durchzuführen und kann leicht automatisiert werden. Wenn paramagnetische oder para- und ferromagnetische Partikel als Festphase verwendet werden, kann zudem ein Zusammenklumpen der Feststoffpartikel weiter vermindert werden. Üblicherweise werden für die magnetische Partikeltechnologie (z. B. der Firma Dynal, Oslo, Norwegen) kleine magnetische Partikel mit einem Durchmesser im nm- oder µm-Bereich verwendet, bei welchen das Problem des Verklumpens besonders stark ist. Die erfindungsgemäße Aufreinigung kann aber ohne Verklumpen durchgeführt werden, da den hydrophoben Gruppen in ausreichender Zahl hydrophile Gruppen, insbesondere Hydroxylgruppen zur Seite gestellt werden.

Die Anbindung von Nukleinsäuren bzw. deren Salzen an besagte Oberfläche wird erfindungsgemäß durch einen Bindepuffer vermittelt. Dieser Bindepuffer enthält ein Salz sowie Polyethylenglykol. Als Salz wird bevorzugt ein Alkali-, Erdalkali- oder/und Ammoniumsalz verwendet, welches als Kation, insbesondere ein Li-, Na-, K-, Rb-, Cs-, Fr-, Be-, Mg-, Ca-, Sr-, Ba-, Raoder/und NH₄-lon enthält. Als Anion enthält das erfindungsgemäß verwendete Salz bevorzugt ein Halogenidanion, insbesondere ein Chloridanion. Das verwendete Polyethylenglykol (PEG) weist bevorzugt eine mittlere Molmasse von 1000 bis 20000 g/Mol, insbesondere von 6000 bis 15000 g/Mol auf.

Da die Viskosität mit steigendem PEG-Gehalt zunimmt, wird dem Bindepuffer bevorzugt ein Alkohol, insbesondere Methanol, Ethanol, Propanol und/oder Butanol, besonders bevorzugt Ethanol oder/und 2-Propanol zugegeben. Der Alkoholgehalt im Bindungspuffer kann bis zu 50 Gew.-% betragen, bevorzugt 30 bis 40 Gew.-%.

Im allgemeinen wird für das erfindungsgemäße Verfahren das Salz bevorzugt in einer Konzentration von ≤ 5 mmol/l, insbesondere von 5 mmol/l bis 4 mol/l, insbesondere bis zu 3 mol/l und das Polyethylenglykol bevorzugt in einer Konzentration von 5 bis 40 %, eingesetzt. Die angegebenen Konzentrationen an Salz und PEG sind finale Konzentrationen und beziehen sich auf die Bindungsbedingungen, d.h. auf das endgültige Gemisch, das Probe und Bindungspuffer und ggf. weitere Verdünnungsmittel, wie etwa Wasser, umfaßt.

Mit dem erfindungsgemäßen Verfahren ist es möglich, DNA über einen großen Molmassenbereich zu immobilisieren und aufzureinigen. Es ist sowohl möglich, nur wenige Basen große Einzelstrang-DNA aufzureinigen als auch mehrere 100 kb große Doppelstrang-DNA, insbesondere BACs, PACs und dgl.

Nukleinsäuren, die mit dem erfindungsgemäßen Verfahren aufgereinigt werden können, umfassen z.B. Einzelstrang-DNA, Doppelstrang-DNA, RNA, LNA sowie Nukleinsäure-Protein-, Nukleinsäure-PNA- und Nukleinsäure-Zucker-Adukte oder Komplexe. Bevorzugt werden Nukleinsäuren immobilisiert, bei denen es sich um Amplifikationsprodukte, beispielsweise aus einer PCR-Reaktion, handelt oder die durch Amplifikation mit Hilfe von Zellen, z.B. mittels einer Übernacht-Kultur, erhalten wurden oder um Sequenzierreaktionsprodukte, Primer-Extensionsreaktionsprodukte, Ligasekettenreaktionsprodukte, Restriktionsendonukleaseverdauprodukte, etc. Es können aber auch synthetisch hergestellte Nukleinsäuren gebunden werden.

Anhand der Konzentration der Bestandteile, insbesondere von Salz und PEG, läßt sich die selektive Anbindung der Einzel- und Doppelstrang-Nukleinsäuren an besagte Oberfläche einstellen. Dabei kann eine Selektivität ≥ 70 %, insbesondere ≥ 80 % und besonders bevorzugt ≥ 90 % erzielt werden. Bei einer Konzentration an einwertigen Kationen von 0,5 bis 4 mol/l, zweiwertigen Kationen ≤ 5 mmol/l und PEG ≤ 15 Gew.-% binden selektiv ds-Nukleinsäuren ≥ 80 bp auch in Gegenwart von ss-Nukleinsäuren. Die Anbindung von ss-Nukleinsäuren gelingt durch Einstellen der Konzentration zweiwertiger Kationen > 5 mmol/l und < 100 mmol/l und PEG von 10 bis 30 Gew.-%. Die Kombination beider Methoden ermöglicht die selektive Aufreinigung von ss- und ds-Nukleinsäuren. Doppelstrang-Nukleinsäuren lassen sich durch Einstellen der Salz- und PEG-Konzentrationen innerhalb der zuvor genannten Bereiche auch nach Größe fraktionieren. Für kleine Nukleinsäuren, insbesondere DNA, hat sich die Verwendung einer finalen Kombination an PEG von 15 - 40 % (Gew./Gew.) und einem Salzgehalt von 10 bis 1000 mmol/l als vorteilhaft erwiesen.

In einer weiteren Ausführungsform betrifft die Erfindung ein Verfahren zur Isolierung oder/und Aufreinigung von Nukleinsäuren umfassend die Schritte
(a) Bereitstellen einer Nukleinsäure enthaltenden Lösung,
(b) Inkontaktbringen der Nukleinsäure enthaltenden Lösung mit einer Festphase, die hydrophobe und hydrophile Gruppen auf der Oberfläche aufweist, in Gegenwart eines Salzes und Polyethylenglykol, wobei die Nukleinsäure reversibel und sequenzunspezifisch über die hydrophoben Gruppen an die Oberfläche gebunden wird,
(c) Abtrennen der Festphase von der Lösung und
(d) gegebenenfalls Ablösen der Nukleinsäure von der Festphase.

Bei der bevorzugten Verwendung einer magnetischen Festphase ist das Abtrennen der Festphase von der Lösung durch magnetische Mittel möglich und kann somit leicht automatisiert werden.

Die Festphase wird bevorzugt einmal vor ihrem Einsatz gewaschen. Abhängig von Anwendung und Oberfläche kann z.B. zum Waschen der mit Wasser (bevorzugt 1:1) verdünnte Bindungspuffer (BP) verwendet werden. Es kann auch eine Lösung von 0,5 mol/l EDTA pH 8 - 9 eingesetzt werden. Wenn eine anschließende Analyse der Probe beabsichtigt ist, kann es sinnvoll sein, die Festphase mit mehreren Puffern zu waschen, u.a. auch mit einem Ammoniumacetat-Puffer.

Eine weitere Verbesserung der Aufreinigung kann dadurch erhalten werden, daß die abgetrennte Festphase, an deren Oberfläche die Nukleinsäure gebunden ist, mit einer Pufferlösung gewaschen wird, welche an die Festphase gebundene Verunreinigungen, nicht aber an die Festphase gebundene Nukleinsäure löst. Die Zusammensetzung der Waschlösung wird in Abhängigkeit der Anwendung und der Oberfläche ausgewählt. Als Waschlösung geeignet ist z.B. 50 - 70 % (vol/vol) Ethanol oder 2-Propanol, ggf. mit Zusätzen von EDTA, CDTA und TRIS in millimolaren Konzentrationen. Falls die Probe anschließend mit Massenspektrometrie analysiert werden soll, ist die Verwendung einer Ammoniumacetat enthaltenden Waschlösung in wenigstens einem Waschschritt bevorzugt, z.B. 0,05 bis 5 mol/l Ammoniumacetat in 60 bis 80 % Ethanol. Oftmals ist es auch vorteilhaft, zur Aufreinigung mehrere Waschschritte mit unterschiedlichen Waschpuffern durchzuführen.

Die auf besagter Oberfläche gebundenen Nukleinsäuremoleküle bzw. deren Salze werden bevorzugt mittels einer Elutionslösung abgetrennt, wobei als Elutionslösung jede Flüssigkeit verwendet werden kann, die die Nukleinsäure wieder von der Festphase ablöst. Die Wahl der Elutionslösung hängt von der Art der verwendeten Oberfläche und des Analyten sowie von der nachfolgenden Verwendung des Analyten ab. Bevorzugt werden als Elutionslösung bidestilliertes Wasser, pH 7 bis 8, eine wäßrige TRIS Lösung mit einer TRIS-Konzentration von 1 bis 100 mmol/l, bevorzugt mit einem pH-Wert von 7 bis 9, eine Formamidlösung, ein Loading Buffer für die Elektrophorese, eine Matrixlösung, z.B. 3-Hydroxypicolinsäure in Wasser (1 - 200 mmol/l) für MALDI-MS etc. verwendet.

Eine magnetische Festphase kann nach Ablösung der Nukleinsäure wiederum mit magnetischen Mitteln abgetrennt werden, wodurch eine vollständige Automatisierung des erfindungsgemäßen Verfahrens erreicht werden kann.

Die mit den erfindungsgemäßen Verfahren isolierten oder/und aufgereinigten Nukleinsäuren können unmittelbar für eine weitere Analyse, z.B. eine Massenspektrometrie (MS)-Analyse verwendet werden. Die bisherige aufwendige manuelle Reinigung über Säulen ist nicht erforderlich. Vielmehr ist es möglich, eine hohe Anzahl von Proben kostengünstig und schnell für eine MS-Analyse, insbesondere eine MALDI-MS-Analyse oder eine ESI (Elektrospray-Ionisation)-MS-Analyse automatisch aufzureinigen. Dabei können insbesondere Begleitstoffe, wie etwa Puffersubstanzen, Metallkationen, Primerüberschüsse, Peptide, Lipide, Detergentien und dgl. entfernt werden. Vorteilhafterweise wird die Nukleinsäure zur Durchführung einer MALDI-MS-Analyse in die Ammoniumform übeführt, z.B. durch einen Ionenaustausch Na⁺ gegen NH₄⁺, der durchgeführt werden kann, während die Nukleinsäuren an die Oberfläche gebunden sind. Die Art der erfindungsgemäßen Anbindung ermöglicht also eine Reinigungsprozedur, d.h. die Nukleinsäure liegt in zugänglicher Form und nicht als Präzipitat vor. Für eine Analyse mit MALDI-MS sind insbesondere kleine DNA-Moleküle von Interesse. Erfindungsgemäß ist die Aufreinigung von kleinen DNA Molekülen oberhalb einer Mindestgröße von ca. 5 Nukleotiden, insbesondere ≥ 10 Nukleotiden möglich. Mit dem erfindungsgemäßen Verfahren werden die bei der MALDI-MS-Analyse störenden Komponenten, wie etwa Puffersubstanzen und Metallkationen sowie evtl. vorhandene Primerüberschüsse effektiv abgetrennt. Die DNA-Moleküle werden zudem in die für eine MALDI-MS-Analyse kompatible Ammoniumform überführt und können wahlweise in reinem Wasser oder in einer wässrigen Matrixlösung eluiert werden und somit direkt auf das MALDI-Target transferiert werden. Eine Modifikation der Zusammensetzung der Bindungs- und Waschpuffer und der sequentielle Einsatz von Partikeln erlaubt es auch, größere DNA-Moleküle von der Aufreinigung auszuschließen, wodurch selektiv ein vorbestimmter Molmassenbereich ausgewählt werden kann, z.B. ≥ 60 bp und ≤ 100 bp.

Durch Variation der Salz- und PEG-Konzentration ist es auch möglich, kleine Oligonukleotide, wie etwa Primer (ssDNA), Primer-Extensionsprodukte, effizient für eine Analyse mit MALDI-MS aufzureinigen.

Ein weiterer Vorteil ist der breite Volumenbereich, in dem mit dem erfindungsgemäßen Verfahren gearbeitet werden kann. Es sind Volumina von ml- bis zum oberen nl-Bereich möglich, insbesondere von < 10 ml, bevorzugt < 1 ml und besonders bevorzugt < 100 µl und ≥ 100 nl, bevorzugt ≥ 1 µl. Der Volumenbereich kann für die jeweilige Applikation eingestellt werden, wobei bei kleinem Volumen eine konzentrierte Probe erhalten wird.

Weiterhin ist es möglich, erfindungsgemäß angebundene Nukleinsäuren nach bekannten Verfahren zu sequenzieren. Die Sequenzierungsbedingungen von bekannten Verfahren stellen oftmals Elutionsbedingungen dar, d.h. während der eigentlichen Sequenzierung ist die Nukleinsäure dann nicht an die Festphase gebunden. In einem solchen Fall ist es vorteilhaft, für die Aufreinigung der Sequenzierprodukte nach Abschlußder Sequenzierreaktion wieder Anbindungsbedingungen, insbesondere durch Zugabe geeigneter Puffer einzustellen. Für die Sequenzierungsreaktion und anschließende Aufreinigung braucht die Festphase, insbesondere Beads, nicht abgetrennt werden. Es hat sich herausgestellt, daß die Festphasen, insbesondere Partikel, oftmals das zur Sequenzierung oftverwendete Thermocycling nicht unbeschadet überstehen. In einem solchen Fall werden nach der Sequenzierreaktion neue, unverbrauchte Beads zugegeben.

Die Erfindung umfaßt weiterhin ein Verfahren zur Synthetisierung von Nukleinsäuren, wobei erfindungsgemäß gebundene Nukleinsäuren nach bekannten Verfahren um mindestens 1 Nukleotid verlängert werden. Ein Beispiel für eine solche Reaktion ist die sog. Primer Extension Reaction, also die Verlängerung eines Primers (ssDNA) um mindestens 1 Nukleotid. Die Verlängerung kann durchgeführt werden, während die Nukleinsäure an die Festphase gebunden ist. Da aber auch bei der Verlängerung von Nukleinsäuren oftmals Bedingungen eingestellt werden, die Elutionsbedingungen entsprechen, kann auch hier das Verlängern stattfinden, während die Nukleinsäure nicht an die Festphase gebunden ist und die Anbindung der verlängerten Nukleinsäure dann anschließend dadurch wieder erreicht werden, daß nach der Verlängerungsreaktion Bindungsbedingungen, beispielsweise durch Pufferzugabe eingestellt werden. Die Festphase braucht während der gesamten Reaktion nicht abgetrennt zu werden.

Die gemäß dem erfindungsgemäßen Verfahren immobilisierten Nukleinsäuren können auch verwendet werden, um selektiv weitere, nachzuweisende Moleküle, beispielsweise Nukleinsäuren oder DNA-bindende Proteine zu binden und können deshalb in entsprechenden Assays eingesetzt werden. Somit umfaßt die Erfindung auch ein Verfahren zum Nachweis eines Analyten in einer Probe, wobei man eine Nukleinsäuren enthaltende Lösung mit einer Festphase, die hydrophobe und hydrophile chemische Gruppen auf der Oberfläche aufweist, in Gegenwart eines Salzes und Polyethylenglykols in Kontakt bringt, wobei die Nukleinsäuren über die hydrophoben Gruppen an die Oberfläche gebunden werden, anschliessend die gebundene Nukleinsäure aufweisende Festphase mit der Probe in Kontakt bringt und den Analyten über die Bindung an die gebundenen Nukleinsäuremoleküle nachweist.

Schließlich umfaßt die Erfindung auch einen Reagenzienkit zur Durchführung des erfindungsgemäßen Verfahrens, der einen Salz und Polyethylenglykol enthaltenden Bindepuffer und eine Festphase, deren Oberfläche hydrophobe und hydrophile Gruppen aufweist. Bevorzugt enthält ein solcher Reagenzienkit zusätzlich Wasch- und Elutionspuffer.

Weiterhin stellt die Erfindung ein Verfahren zum Anbinden von Nukleinsäuren an eine Festphase bereit, welches dadurch gekennzeichnet ist, daß eine Nukleinsäuren enthaltende Lösung mit einer Festphase, welche eine hydrophile wasserhaltige Polymermatrix umfaßt, in Gegenwart von wasserentziehenden Reagenzien in Kontakt gebracht wird, wobei die Nukleinsäuren reversibel und sequenzunspezifisch an die Festphase gebunden werden.

Es wurde überraschenderweise festgestellt, daß durch die Verwendung von hydrophilen wasserhaltigen Polymermatrices eine große Bindekapazität im Hinblick auf Nukleinsäuren erhalten werden kann und auch die Isolierung von sehr kleinen DNA-Fragmenten, beispielsweise mit einer Länge von 10 bis 100 Bp, insbesondere von 30 bis 70 Bp durchgeführt werden kann. Zudem ist in Abhängigkeit der Auswahl der hydrophilen wasserhaltigen Polymermatrix eine größenselektive Bindung von Nukleinsäuren möglich.

Bevorzugt wird beim erfindungsgemäßen Verfahren zuerst die hydrophile wasserhaltige Polymermatrix mit einer Nukleinsäuren enthaltenden Lösung in Kontakt gebracht und in einem zweiten Schritt dann ein wasserentziehendes Mittel zugegeben. Geeignete wasserentziehende Mittel sind Salze, insbesondere chaotrope Salzpuffer mit hoher Konzentration, sowie Polyethylenglykol. Bevorzugte Salze und Polyethylenglykole sowie deren Konzentration sind wie oben beschrieben.

Durch die Verwendung einer hydrophilen wasserhaltigen Polymermatrix als Festphasenmaterial anstelle von Silika oder Borsilikatglas, wie es üblicherweise verwendet wird, kann eine Bindung von Nukleinsäuren, welche reversibel und sequenzunspezifisch ist, erhalten werden. Die Polymermatrix enthält bevorzugt von 1 bis 90, mehr bevorzugt von 10 bis 50 Gew.-% Wasser.

Die hydrophile wasserhaltige Polymermatrix enthält bevorzugt ein hydrophiles wasserlösliches Polymer, insbesondere ein hydrophiles wasserlösliches organisches Polymer. Besonders geeignet sind Polysaccharide, insbesondere Polysaccharide mit endständigen Hydroxylgruppen, wie etwa Dextran oder Stärke. Besonders bevorzugt ist Dextran. Die hydrophile wasserhaltige Polymermatrix wird besonders vorteilhaft als Hüllpolymer um einen magnetischen Kern bereitgestellt, beispielsweise in Form von Dextran-Magnetitparikeln.

Die hydrophile wasserhaltige Polymermatrix kann auf ihrer Oberfläche hydrophile oder/und hydrophobe Gruppen enthalten. Bevorzugt ist eine Ausführungsform, in der eine Festphase verwendet wird, die sowohl hydrophobe als auch hydrophile Gruppen, wie oben beschrieben, auf der Oberfläche aufweist und gleichzeitig eine hydrophile wasserhaltige Polymermatrix umfaßt.

Eine Festphase mit einer hydrophilen wasserhaltigen Polymermatrix kann in gleicher Weise wie oben beschrieben in einem Verfahren zur Isolierung oder/und Aufreinigung von Nukleinsäuren oder in einem Verfahren zum Nachweis eines Analyten in einer Probe eingesetzt werden. Darüber hinaus kann sie zur Bestimmung der Nukleinsequenz einer Nukleinsäure oder zur Synthetisierung von Nukleinsäuren herangezogen werden.

Das erfindungsgemäße Verfahren wird durch die beigefügten Figuren und folgenden Beispiele weiter erläutert.

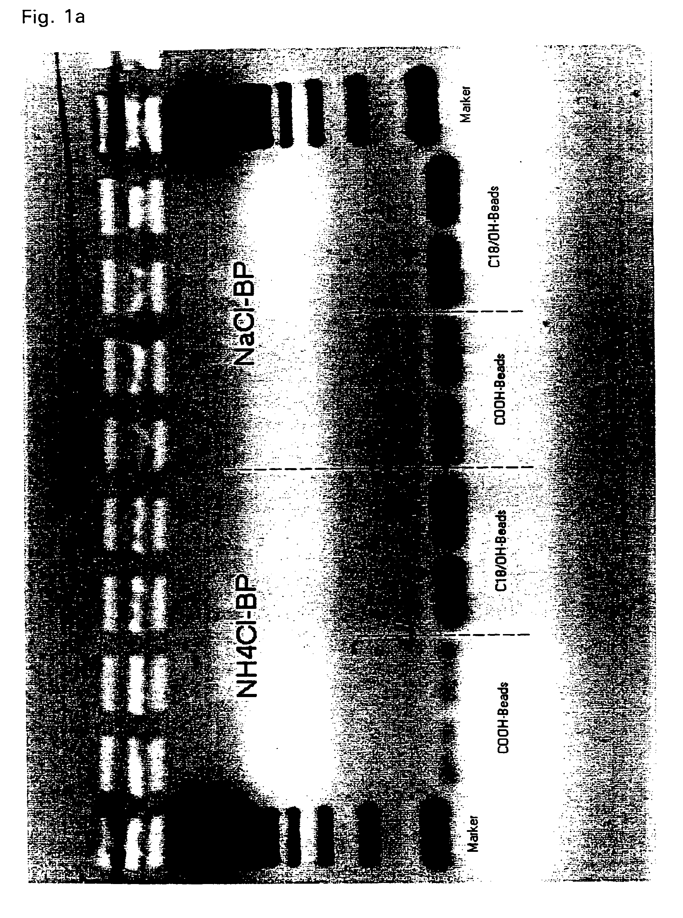

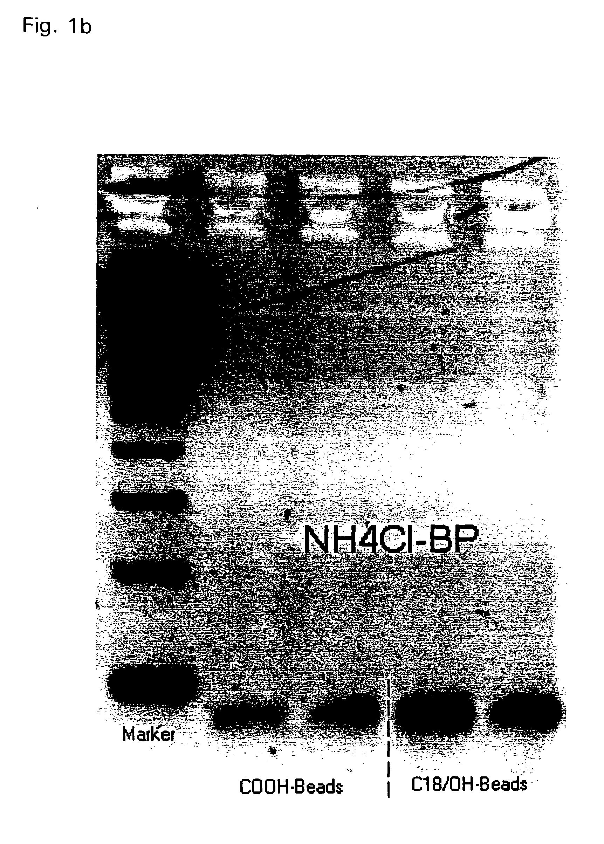

- Figur 1: (bestehend aus Fig.1a und 1b) zeigt ein Agarosegel von erfindungsgemäß aufgereinigten PCR-Produkten sowie von PCR-Produkten, die unter Verwendung von COOH-beschichteten Partikeln aufgereinigt wurden:
- Figur 1a: zeigt einen Ausbeutevergleich unter Verwendung eines NH₄Cl-Bindepuffers (links) sowie eines NaCl-Bindepuffers (rechts) für erfindungs gemäßaufgereinigte PCR-Produkte (C18/OH-Beads) sowie für PCR-Produkte, die unter Verwendung von COOH-beschichteten Partilen aufgereinigt wurden (COOH-Beads).
- Figur 1 b: zeigt einen weiteren Ausbeutevergleich mit NH₄Cl-Bindepuffer.
- Figur 2: (bestehend aus Fig. 2a, 2b und 2c) zeigt die Isolierung und Aufreinigung von ssDNA und dsDNA mit dem erfindungsgemäßen Verfahren für eine MALDI-MS-Analytik:
- Figur 2a: zeigt das MALDI-Flugzeitmassenspektrum von erfindungsgemäß aufgereinigten PCR-Produkten mit 47 bzw. 48 Basenpaaren.
- Figur 2b: zeigt das MALDI-Flugzeitmassensepktrum eines erfindungsgemäß aufgereinigten PCR-Produktes mit 80 bp.
- Figur 2c: zeigt das MALDI-Flugzeitmassenspektrum eines erfindungsgemäß aufgereinigten 24 Nukleotide langen DNA Einzelstrangs.

Bei MALDI werden bevorzugt einfach geladene Molekülionen der Spezies (M + H) ⁺, und daneben mit verringerter Häufigkeit auch doppelt geladene Molekülionen der Spezies (M + 2H)²⁺ gebildet. PCR-Produkte werden bei der MALDI-Massenspektrometrie, sofern nicht besondere Maßnahmen ergriffen werden, aufgetrennt und in Form der Einzelstränge detektiert. Die Signale zueinander komplementärer Einzelstränge werden oft aufgrund geringer Massenunterschiede nur partiell aufgelöst. Die Größe der PCR-Produkte läßt sich dennoch durch Vergleich der gemessenen mittleren Massen mit abgeschätzten oder aufgrund von bekannten Sequenzen berechneten Werten bestimmen. Die (M + H)⁺-Signale nicht abgetrennter Primer würden in den Spektren an den mit den Pfeilen gekennzeichneten Positionen registriert werden.

### Beispiele

### Beispiel 1 dsDNA-Isolierung und Aufreinigung aus PCR (Polymerasekettenreaktion)

Magnetische Partikel, deren Oberfläche hydrophobe und hydrophile Gruppen aufweist, werden zunächst dreimal mit 150 µl EDTA-Lösung (0,5 mol/l, pH 8) durch magnetische Separation der Partikel und Verwerfen des Überstandes gewaschen. Nach dem letzten Waschen werden die Partikel in EDTA-Lösung aufgenommen und vermischt. Die Massenkonzentration beträgt 20 mg/ml.

Die zu untersuchenden Proben werden in einer Mikrotiterplatte (z.B. 96 well) vorgelegt. Zu 40 µl Probevolumen werden 40 µl Bindungspuffer (2,5 mol/l NaCl, 20% (Gew./Gew.) PEG6000) und 10 µl der Partikelsuspension zugegeben, vermischt und bei Raumtemperatur 10 min inkubiert.

Die Mikrotiterplatte mit den Proben wird anschließend für 2 min in eine Magnethalterung gestellt, der Überstand verworfen und die Partikel zweimal mit 150 µl Waschpuffer (40% Ethanol) gewaschen und anschließend an Luft 2 bis 5 min getrocknet.

Schließlich wird die Mikrotiterplatte aus dem Magnethalter entnommen und die Partikel in 20 ml Elutionspuffer (1 mmol/l Tris-HCL) resuspendiert und 5 min inkubiert. Die Mikrotiterplatte wird dann wiederum in die Magnethalterung gestellt und das Eluat nach 2 min abgenommen.

Die im Eluat enthaltene DNA konnte ohne weitere Aufreinigung für Konzentrationsbestimmungen, zu DNA-Sequenzierung usw. verwendet werden.

### Beispiel 2 dsDNA-Isolierung und Aufreinigung aus Zellkultur

Zellen aus einer Übernachtkultur wurden pelletiert und der Überstand verworfen. Das Zellpellet wurde in 40 µl Resuspendierungspuffer (50 mmol/l Tris-HCl, pH 8, 10 mmol/l EDTA, 100 µg/ml RNAse A) aufgenommen und vermischt. Dann wurden 40 µl Lysepuffer (200 mmol/l NAOH, 1% (Gew./Gew.) SDS) zugegeben und vermischt. Nach Zugabe von 40 µl Neutralisierungspuffer (3 mol/l KOAc, pH 5,5) und Vermischen wurde 15 min bei 13.000 U/min zentrifugiert.

Der Überstand wurde in eine frische Mikrotiterplatte transferiert. Erfindungsgemäße magnetische Partikel wurden dreimal mit 150 µl EDTA-Lösung (0,5 mol/l pH 8) durch magnetische Separation der Partikel und Verwerfen des Überstandes gewaschen. Nach dem letzten Waschen wurden die Partikel in EDTA-Lösung aufgenommen und vermischt. Die Massekonzentration betrug 20 mg/ml.

Zu 120 µl Probevolumen wurden 120 µl Bindungspuffer (2,5 mol/l NaCl, 20% (Gew./Gew.) PEG 6000) und 10 µl der Partikelsuspension zugegeben. Nach Mischen des Ansatzes wurde 5 min bei Raumtemperatur inkubiert.

Die Mikrotiterplatte mit den Proben wurde anschließend für 10 min in eine Magnethalterung gestellt, der Überstand verworfen und die Partikel zweimal mit 120 µl Waschpuffer (70% Ethanol, 10 mmol/l Tris-HCl, pH 8, 1 mmol/l EDTA) gewaschen und anschließend an der Luft 5 min getrocknet.

Die Mikrotiterplatte wurde dann aus dem Magnethalter genommen und die Partikel in 50 µl Elutionspuffer (1 mmol/l Tris-HCL, pH 8) resuspendiert, 5 min inkubiert und die Platte dann wieder in den Magnethalter gestellt und das Eluat nach 2 min abgenommen.

Die im Eluat enthaltene DNA konnte ohne weitere Aufreinigung für Konzentrationsbestimmungen, DNA-Sequenzierung usw. verwendet werden.

### Beispiel 3 Ausbeutevergleich

Figur 1 zeigt das Agarosegel von aufgereinigten 100 bp PCR-Produkten. Die Aufreinigung wurde zum einen gemäß der Erfindung und zum anderen unter Verwendung von COOH-beschichteten Partikeln (vgl. US-Patent 5,705,628) durchgeführt. Den PCR-Produkten wurden vor der Aufreinigung 100 pmol einer 32 Nukleotide großen ssDNA zugegeben. Wie man aus der Abbildung des Agarose-Gels sehen kann, wurde die gewünschte Nukleinsäure in gereinigter Form erhalten und unerwünschte Substanzen inklusive der 32 Nukleotide ssDNA abgetrennt. Weiterhin ist die Ausbeute beim erfindungsgemäßen Verfahren deutlich höher als den COOH-beschichteten Partikeln.

### Beispiel 4 Aufreinigungsprotokolle

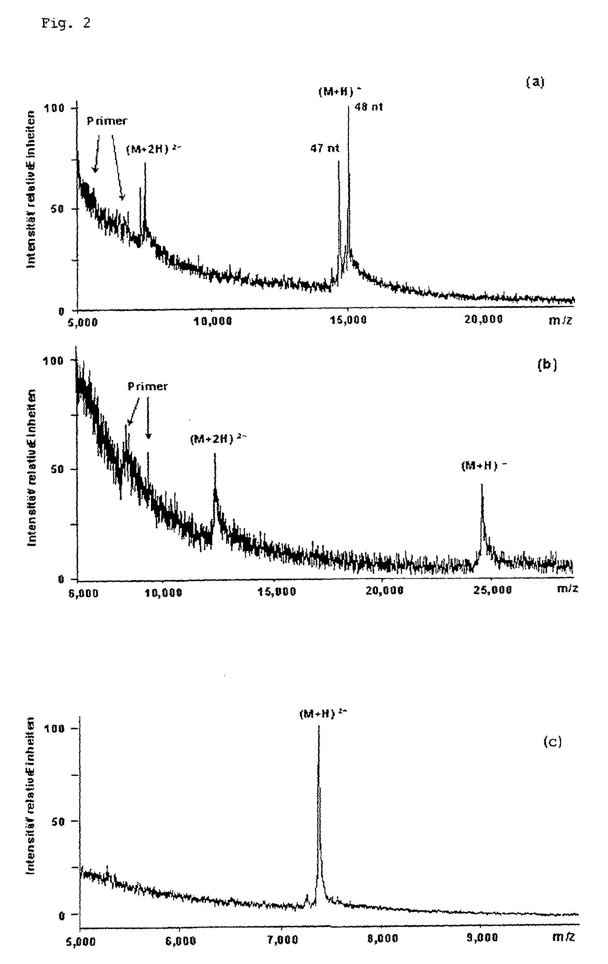

Zur Aufreinigung von Doppelstrang DNA (vgl. Figur 2a und 2b) wurden PCR-Reaktionen jeweils in 40 µl Reaktionsvolumina in 96iger Titerplatten durchgeführt. Magnetische Partikel wurden vor Gebrauch magnetisch separiert, wobei überstehende Lösung abpipetiert und die Partikel in einem Aliquot des Bindungspuffers resuspendiert wurden. Nach Abschluß der Amplifikationsreaktion wurden zu jedem Reaktionsgefäß 5 µl der Suspension der magnetischen Partikel zugegeben, gefolgt von 50 µl Bindungspuffer und zwar für die PCR-Produkte mit 47/48 bp (vgl. Figur 2a): 1,5 M NH₄Cl/40 mM MgCl₂ in 40 % (Gew./Gew.) PEG 6000/60% (Gew./Gew.) H₂O und für das PCR Produkt mit 80 bp: 2,5 M NH4Cl in 30 % (Gew./-Gew.) PEG 6000/70% (Gew./Gew.) H₂O.

Die resultierende Suspension wurde gut durchmischt und 10 min stehengelassen. Danach wurden die Partikel magnetisch separiert, der Überstand abpipetiert und durch 105 µl 65 % (vol/vol) Ethanol/35 % (vol/vol) H₂O (Waschlösung 1) ersetzt. Durch Umsetzen der Gefäße wurden die magnetischen Partikel zweimal durch die Waschlösung bewegt. Anschließend wurde der Überstand nacheinander durch 115, 125 und 135 µl 1,5 M Ammoniumacetat in 30 % (vol/vol) H₂O, 70 % (vol/vol) Ethanol ersetzt, wobei die Partikel jeweils fünfmal durch die neue Waschlösung 2 bewegt wurden. Die Überstände wurden jeweils verworfen. Schließlich wurden die Partikel zweimal mit 145 µl 80 % (vol/vol) Ethanol/20 % (vol/vol) H₂O (Waschlösung 3) gewaschen, wobei die Partikel jeweils zweimal durch die Lösung bewegt wurden. Nach Abpipettieren des letzten Überstandes wurden die Partikel zum Abdampfen des verbliebenen Ethanols 10 min an Luft stehen gelassen. Anschließend wurden die Partikel in 5 µl 1 mM Tris-HCl, pH 7,5 (Elutionslösung) resuspendiert. Nach 10 min wurden die Partikel magnetisch abgetrennt und 0,5 µl des Überstandes auf einen frischgereinigten MALDI-Probenträger überführt und dort mit 0,5 µl Matrixlösung (200 mM 3-Hydroxypicolinsäure in 30 % (vol/vol) Acetonitril/70 % H₂O (vol/vol)) vermischt. Nach Abdampfen des Lösungsmittels wurde die Probe in einen Bruker Reflex II MALDI-Flugzeitmassenspektrometer analysiert.

Zur Aufreinigung der Einzelstrang-DNA (vgl. Figur 2c) wurden 10 pmol eines 24 Nukleotide langen Oligodeoxyribonukleotids in 40 µl PCR-Reaktionslösung vorgelegt und hierzu 5 µl der Suspension der magnetischen Partikel (in Bindepuffer) gegeben, gefolgt von 50 µl Bindepuffer (1,5 M NH₄Cl/40 mM MgCl₂ in 35 % (Gew./Gew.) PEG 6000/30 % (vol/vol) Ethanol. Die Waschprozedur unterschied sich von der obigen darin, daß die erste Waschung ausgelassen wurde und die Waschlösung 2 durch 100 mM Ammoniumacetat in 30% (vol/vol) H₂O/70% (vol/vol) Ethanol ersetzt wurde. Die Elution und anschließende Analyse des aufgereinigten Produkts wurden wie oben beschrieben durchgeführt.

## Patentansprüche

1. Verfahren zum Anbinden von Nukleinsäuren an eine Festphase,
**dadurch gekennzeichnet,**
**daß** eine Nukleinsäuren enthaltende Lösung mit einer Festphase, die hydrophobe und hydrophile Gruppen auf der Oberfläche aufweist, in Gegenwart eines Salzes und Polyethylenglykols in Kontakt gebracht wird, wobei die Nukleinsäuren reversibel und sequenzunspezifisch über die hydrophoben Gruppen an die Oberfläche gebunden werden.

2. Verfahren nach Anspruch 1,
**dadurch gekennzeichnet,**
**daß** besagte Oberfläche Alkyl- oder Arylgruppen als hydrophobe Gruppen aufweist.

3. Verfahren nach Anspruch 2,
**dadurch gekennzeichnet,**
**daß** die Alkylgruppen ausgewählt werden aus C₈-Alkyl, C₁₈-Alkyl und Mischungen davon.

4. Verfahren nach einem der Ansprüche 1 bis 3,
**dadurch gekennzeichnet,**
**daß** die Oberfläche Hydroxylgruppen als hydrophile Gruppen aufweist.

5. Verfahren nach einem der vorhergehenden Ansprüche,
**dadurch gekennzeichnet,**
**daß** es sich bei der Festphase um Festpartikel handelt.

6. Verfahren nach einem der vorhergehenden Ansprüche,
**dadurch gekennzeichnet,**
**daß** die Festphase magnetisch ist.

7. Verfahren nach einem der vorhergehenden Ansprüche,
**dadurch gekennzeichnet,**
**daß** es sich bei dem Salz um ein Alkali-, Erdalkali- oder/und Ammoniumhalogenid handelt.

8. Verfahren nach einem der vorhergehenden Ansprüche,
**dadurch gekennzeichnet,**
**daß** ein Polyethylenglykol mit einer mittleren Molmasse von 1000 bis 20000 g/mol zugegeben wird.

9. Verfahren nach einem der vorhergehenden Ansprüche,
**dadurch gekennzeichnet,**
**daß** das Salz in einer finalen Konzentration von 5 mmol/l bis 4 mol/l verwendet wird.

10. Verfahren nach einem der vorhergehenden Ansprüche,
**dadurch gekennzeichnet,**
**daß** Polyethylenglykol in einer finalen Konzentration von 5 Gew. % bis 40 Gew.-% verwendet wird.

11. Verfahren nach einem der vorhergehenden Ansprüche,
**dadurch gekennzeichnet,**
**daß** es sich bei der Nukleinsäure um DNA handelt.

12. Verfahren nach einem der vorhergehenden Ansprüche,
**dadurch gekennzeichnet,**
**daß** es sich bei der Nukleinsäure um Amplifikationsprodukte handelt.

13. Verfahren nach einem der vorhergehenden Ansprüche,
**dadurch gekennzeichnet,**
**daß** selektiv Einzelstrang- oder Doppelstrang-Nukleinsäuren gebunden werden.

14. Verfahren nach einem der vorhergehenden Ansprüche,
**dadurch gekennzeichnet,**
**daß** die Nukleinsäure in einem Bereich von ≥ 5 Nukleotide bis ≤ 1000 Nukleotide selektiv hinsichtlich der Größe gebunden wird.

15. Verfahren zur Isolierung oder/und Aufreinigung von Nukleinsäuren umfassend die Schritte
(a) Bereitstellen einer Nukleinsäure enthaltenden Lösung,
(b) Inkontaktbringen der Nukleinsäure enthaltenden Lösung mit einer Festphase, die hydrophobe und hydrophile Gruppen auf der Oberfläche aufweist, in Gegenwart eines Salzes und Polyethylenglykol, wobei die Nukleinsäure reversibel und sequenzunspezifisch über die hydrophoben Gruppen an die Oberfläche gebunden wird,
(c) Abtrennen der Festphase von der Lösung und
(d) gegebenenfalls Ablösen der Nukleinsäure von der Festphase.

16. Verfahren nach Anspruch 15,
**dadurch gekennzeichnet,**
**daß** die Festphase magnetisch ist und das Abtrennen der Festphase von der Lösung durch magnetische Mittel erfolgt.

17. Verfahren nach Anspruch 15 oder 16,
**dadurch gekennzeichnet,**
**daß** die in Schritt (c) abgetrennte Festphase mit einer Pufferlösung gewaschen wird, welche an die Festphase gebundene Verunreinigungen, nicht aber an die Festphase gebundene Nukleinsäure löst.

18. Verfahren nach einem der Ansprüche 15 bis 17,
**dadurch gekennzeichnet,**
**daß** das Ablösen der Nukleinsäure in Schritt (d) mittels einer Elutionslösung durchgeführt wird.

19. Verfahren nach einem der Ansprüche 15 bis 18,
**dadurch gekennzeichnet,**
**daß** von der Festphase abgelöste Nukleinsäure und die Festphase mit magnetischen Mitteln getrennt werden.

20. Verfahren nach einem der Ansprüche 15 bis 19,
**dadurch gekennzeichnet,**
**daß** die gewonnene Nukleinsäure einer massenspektrometrischen Analyse unterzogen wird.

21. Verfahren zur Bestimmung der Nukleotidsequenz einer Nukleinsäure umfassend die Schritte:
(a) Anbinden einer Nukleinsäure an eine Festphase gemäß dem Verfahren nach Anspruch 1 und
(b) Sequenzieren der Nukleinsäure nach bekannten Verfahren.

22. Verfahren nach Anspruch 21, weiterhin umfassend den Schritt
(c) Aufreinigung der Sequenzierungsprodukte. ,

23. Verfahren zur Synthetisierung von Nukleinsäuren umfassend die Schritte
(a) Anbinden einer Nukleinsäure an eine Festphase gemäß dem Verfahren nach Anspruch 1 und
(b) Verlängern der Nukleinsäure um mindestens ein Nukleotid nach bekannten Verfahren.

24. Verfahren zum Nachweis eines Analyten in einer Probe,
**dadurch gekennzeichnet,**
**daß** man eine Nukleinsäuren enthaltende Lösung mit einer Festphase, die hydrophobe und hydrophile Gruppen auf der Oberfläche aufweist, in Gegenwart eines Salzes und Polyethylenglykols in Kontrakt bringt, wobei die Nukleinsäuren reversibel und sequenzunspezifisch über die hydrophoben Gruppen an die Oberfläche gebunden werden, anschließend diese Festphase mit der Probe in Kontakt bringt und den Analyten über die Bindung an die gebundenen Nukleinsäuren nachweist.

25. Reagenzienkit zur Durchführung eines Verfahrens gemäß einem der Ansprüche 1 bis 24 umfassend:
(a) einen Bindepuffer, der ein Salz und ein Polyethylenglykol enthält und
(b) eine Festphase, die hydrophobe und hydrophile Gruppen auf der Oberfläche aufweist.

26. Reagenzienkit nach Anspruch 25,
weiterhin umfassend,
(c) einen Elutionspuffer, mit dem an diese Oberfläche gebundene Nukleinsäure abgelöst werden können,
(d) einen Waschpuffer, mit dem an die Festphase gebundene Verunreinigungen abgetrennt werden können.

## Claims

1. Method for binding nucleic acids to a solid phase
**characterized in that**
a solution containing nucleic acids is contacted with a solid phase which has hydrophobic and hydrophilic groups on its surface in the presence of a salt and polyethylene glycol, whereby the nucleic acids are reversibly and sequence-unspecifically bound to the surface by the hydrophobic groups.

2. Method according to claim 1,
**characterized in that**
the said surface has alkyl or aryl groups as hydrophobic groups.

3. Method according to claim 2,
**characterized in that**
the alkyl groups are selected from C₈ alkyl, C₁₈ alkyl and mixtures thereof.

4. Method according to one of the claims 1 to 3,
**characterized in that**
the surface has hydroxyl groups as hydrophilic groups.

5. Method according to one of the previous claims,
**characterized in that**
the solid phase comprises solid particles.

6. Method according to one of the previous claims,
**characterized in that**
the solid phase is magnetic.

7. Method according to one of the previous claims,
**characterized in that**
the salt is an alkali, alkaline earth or/and ammonium halide.

8. Method according to one of the previous claims,
**characterized in that**
a polyethylene glycol having an average molar mass of 1000 to 20000 g/mol is added.

9. Method according to one of the previous claims,
**characterized in that**
the salt is used at a final concentration of 5 mmol/l to 4 mol/l.

10. Method according to one of the previous claims,
**characterized in that**
polyethylene glycol is used at a final concentration of 5 % by weight to 40 % by weight.

11. Method according to one of the previous claims,
**characterized in that**
the nucleic acid is DNA.

12. Method according to one of the previous claims,
**characterized in that**
the nucleic acid is an amplification product.

13. Method according to one of the previous claims,
**characterized in that**
single-stranded and double-stranded nucleic acids are selectively bound.

14. Method according to one of the previous claims,
**characterized in that**
the nucleic acid is selectively bound with regard to size in a range of ≥ 5 nucleotides to ≤ 1000 nucleotides.

15. Method for isolating or/and purifying nucleic acids comprising the steps
(a) preparing a solution containing nucleic acids,
(b) contacting the solution containing nucleic acids with a solid phase which has hydrophobic and hydrophilic groups on its surface in the presence of a salt and polyethylene glycol whereby the nucleic acid is reversibly and sequence-unspecifically bound to the surface by the hydrophobic groups,
(c) separating the solid phase from the solution and
(d) optionally detaching the nucleic acid from the solid phase.

16. Method according to claim 15,
**characterized in that**
the solid phase is magnetic and the solid phase is separated from the solution by magnetic means.

17. Method according to claim 15 or 16,
**characterized in that**
the solid phase separated in step (c) is washed with a buffer solution which detaches impurities bound to the solid phase but not the nucleic acids bound to the solid phase.

18. Method according to one of the claims 15 to 17,
**characterized in that**
the-nucleic acid is detached in step (d) by means of an elution solution.

19. Method according to one of the claims 15 to 18,
**characterized in that**
the nucleic acid detached from the solid phase and the solid phase are separated by magnetic means.

20. Method according to one of the claims 15 to 19,
**characterized in that**
the nucleic acid obtained is subjected to a mass spectrometric analysis.

21. Method for the determining the nucleotide sequence of a nucleic acid comprising the steps:
(a) binding a nucleic acid to a solid phase according to the method of claim 1 and
(b) sequencing the nucleic acid by known methods.

22. Method according to claim 21, additionally comprising the step
(c) purifying the sequencing products.

23. Method for synthesizing nucleic acids comprising the steps
(a) binding a nucleic acid to a solid phase according to the method of claim 1 and
(b) extending the nucleic acid by at least one nucleotide by known methods.

24. Method for detecting an analyte in a sample,
**characterized in that**
a solution containing nucleic acids is contacted with a solid phase which has hydrophobic and hydrophilic groups on its surface in the presence of a salt and polyethylene glycol whereby the nucleic acids are reversibly and sequence-unspecifically bound to the surface by the hydrophilic groups, subsequently this solid phase is contacted with the sample and the analyte is detected by means of the binding to the bound nucleic acids.

25. Reagent kit for-carrying out a method according to one of the claims 1 to 24 comprising:
(a) a binding buffer which contains a salt and a polyethylene glycol and
(b) a solid phase which has hydrophobic and hydrophilic groups on its surface.

26. Reagent kit according to claim 25,
additionally comprising,
(c) an elution buffer that can be used to detach the nucleic acid bound to this surface,
(d) a washing buffer which can be used to separate impurities bound to the solid phase.

## Revendications

1. Procédé de liaison d'acides nucléiques à une phase solide, **caractérisé en ce qu'**une solution contenant des acides nucléiques est mise en contact avec une phase solide qui présente, à sa surface, des groupes hydrophobes et hydrophiles, en présence d'un sel et de polyéthylène glycol, les acides nucléiques étant liés à la surface de façon réversible et non spécifique d'une séquence par les groupes hydrophobes.

2. Procédé selon la revendication 1, **caractérisé en ce que** ladite surface présente des groupes alkyle ou aryle en tant que groupes hydrophobes.

3. Procédé selon la revendication 2, **caractérisé en ce que** les groupes alkyle sont choisis parmi les groupes alkyle en C₈, alkyle en C₁₈ et leurs mélanges.

4. Procédé selon l'une des revendications 1 à 3, **caractérisé en ce que** la surface présente des groupes hydroxyle en tant que groupes hydrophiles.

5. Procédé selon l'une des revendications précédentes, **caractérisé en ce que** la phase solide est constituée de particules solides.

6. Procédé selon l'une des revendications précédentes, **caractérisé en ce que** la phase solide est magnétique.

7. Procédé selon l'une des revendications précédentes, **caractérisé en ce que** le sel est un halogénure d'un métal alcalin, d'un métal alcalino-terreux et/ou d'ammonium.

8. Procédé selon l'une des revendications précédentes, **caractérisé en ce que** l'on ajoute un polyéthylèneglycol présentant une masse molaire mo yenne de 1000 à 20000 g/mole.

9. Procédé selon l'une des revendications précédentes, **caractérisé en ce que** le sel est utilisé en une concentration finale de 5 mmoles/l à 4 moles/l.

10. Procédé selon l'une des revendications précédentes, **caractérisé en ce que** le polyéthylèneglycol est utilisé en une concentration finale de 5 % en poids à 40 % en poids.

11. Procédé selon l'une des revendications précédentes, **caractérisé en ce que** l'acide nucléique est un ADN.

12. Procédé selon l'une des revendications précédentes, **caractérisé en ce que** l'acide nucléique est constitué de produits d'amplification.

13. Procédé selon l'une des revendications précédentes, **caractérisé en ce que** des acides nucléiques à brin simple ou à double brin sont liés sélectivement.

14. Procédé selon l'une des revendications précédentes, **caractérisé en ce que** l'acide nucléique est lié dans une plage de ≥ 5 nucléotides à ≤ 1000 nucléotides de façon sélective eu égard à sa taille.

15. Procédé d'isolement et/ou de purification d'acides nucléiques, comprenant les étapes consistant à
(a) préparer une solution contenant un acide nucléique
(b) mettre en contact la solution contenant l'acide nucléique avec une phase solide qui présente, à sa surface, des groupes hydrophobes et hydrophiles, en présence d'un sel et de polyéthylène glycol, l'acide nucléique étant lié à la surface de façon réversible et non spécifique d'une séquence par les groupes hydrophobes,
(c) séparer la phase solide de la solution et
(d) éventuellement, détacher l'acide nucléique de la phase solide.

16. Procédé selon la revendication 15, **caractérisé en ce que** la phase solide est magnétique et que la séparation de la phase solide de la solution s'effectue par un moyen magnétique.

17. Procédé selon la revendication 15 ou 16, **caractérisé en ce que** la phase solide séparée à l'étape (c) est lavée avec une solution tampon qui dissout les impuretés liées à la phase solide mais non les acides nucléiques liés à la phase solide.

18. Procédé selon l'une des revendications 15 à 17, **caractérisé en ce que** la séparation de l'acide nucléique à l'étape (d) s'effectue au moyen d'une solution d'élution.

19. Procédé selon l'une des revendications 15 à 18, **caractérisé en ce que** l'acide nucléique détaché de la phase solide et la phase solide sont séparés avec des moyens magnétiques.

20. Procédé selon l'une des revendications 15 à 19, **caractérisé en ce que** l'acide nucléique obtenu est soumis à une analyse de spectrométrie de masse.

21. Procédé de détermination de la séquence de nucléotides d'un acide nucléique comprenant les étapes consistant à :
(a) lier un acide nucléique à une phase solide selon le procédé de la revendication 1 et
(b) séquencer l'acide nucléique selon des procédés connus.

22. Procédé selon la revendication 21, comprenant en outre l'étape consistant à
(c) purifier les produits de séquençage.

23. Procédé de synthèse d'acides nucléiques comprenant les étapes consistant à :
(a) lier un acide nucléique à une phase solide selon le procédé de la revendication 1 et
(b) prolonger l'acide nucléique d'au moins un nucléotide selon des procédés connus.

24. Procédé de détection d'un analyte dans un échantillon, **caractérisé en ce que** l'on met en contact une solution contenant des acides nucléiques avec une phase solide qui présente, à sa surface, des groupes hydrophobes et hydrophiles, en présence d'un sel et de polyéthylèneglycol, les acides nucléiques étant liés sur la surface de façon réversible et non spécifique d'une séquence par les groupes hydrophobes, **en ce que** l'on soumet ensuite cette phase solide en contact avec l'échantillon et **en ce que** l'on détecte l'analyte par la liaison aux acides nucléiques liés.

25. Kit de réactifs pour réaliser un procédé selon l'une des revendications 1 à 24, comprenant :
(a) un tampon de liaison qui contient un sel et un polyéthylèneglycol et
(b) une phase solide qui présente, à sa surface, des groupes hydrophobes et hydrophiles.

26. Kit de réactif selon la revendication 25, comprenant en outre
(c) un tampon d'élution avec lequel les acides nucléiques liés à cette surface peuvent être détachés,
(d) un tampon de lavage avec lequel les impuretés liées à une phase solide peuvent être séparées.
